# EUROPEAN PATENT APPLICATION

(11) **EP 4 671 377 A1**
(43) Date of publication of application: **31.12.2025**
(21) Application number: 24760265.9
(22) Date of filing: 16.02.2024
(51) Int. Cl.: C12P 23/00, A23L 33/10, A61K 8/49, A61K 8/9706, A61Q 19/00, C12N 1/12, C07D 303/32

(54) **PRODUCTION METHOD FOR FUCOXANTHIN-CONTAINING COMPOSITION, FUCOXANTHIN-CONTAINING COMPOSITION, USE FOR FUCOXANTHIN-CONTAINING COMPOSITION, DIATOM**

(30) Priority: 22.02.2023 JP 2023026634
(71) Applicant: National University Corporation Toyohashi University of Technology, Tempaku-cho, Toyohashi-shi, Aichi 441-8580 (JP)
(72) Inventor: HIROSE Yuu, Toyohashi-shi, Aichi 441-8580 (JP)
(74) Representative: Kuhnen & Wacker Patent- und Rechtsanwaltsbüro PartG mbB
(86) International application number: PCT/JP2024/005492
(87) International publication number: WO 2024/176965

(57) **Abstract**

Provided is a technology capable of improving antimicrobial properties in a fucoxanthin-containing composition. A method for producing a fucoxanthin-containing composition includes the step of culturing a diatom in a medium at pH 1.0 or more and pH 4.0 or less, wherein the diatom has 18S rRNA composed of the following (a) or (b): (a) a polynucleotide consisting of a nucleotide sequence represented by SEQ ID NO: 1; or (b) a polynucleotide consisting of a nucleotide sequence having 85% or more identity to the nucleotide sequence represented by SEQ ID NO: 1.

## Description

### [Technical Field]

The present disclosure relates to the production of a fucoxanthin-containing composition. The present application is based on Japanese Patent Application No. 2023-026634, filed on February 22, 2023, the contents of which are herein incorporated by reference.

### [Background Art]

Fucoxanthin is a type of carotenoid contained in large brown algae such as kelp and wakame, as well as in microalgae including diatoms, haptophytes, raphidophytes, and dinoflagellates. It is known that fucoxanthin has excellent efficacy such as an antioxidant action and an antiobesity action (e.g., NPL 1). Fucoxanthin may be produced using diatoms. For example, PTL 1 discloses a method for culturing diatoms by irradiation with green light.

### [Citation List]

### [Patent Literature]

[PTL 1] JP 2020-074733 A

### [Non Patent Literature]

[NPL 1] Sho Nishikawa and two others, "Anti-obesity/anti-diabetes effects of brown algae-derived fucoxanthin and their mechanisms", Kagaku to Seibutsu "Chemistry and Biology", 2016, Vol. 54, No. 8, p. 580-585.

### [Summary of Invention]

### [Technical Problem]

The fucoxanthin-containing algae obtained by the method described in PTL 1 have room for improvement from the viewpoint of antimicrobial properties. For this reason, a technology has been sought to be able to improve antimicrobial properties in a fucoxanthin-containing composition.

### [Solution to Problem]

The present invention can be implemented in the following items.

(1) An aspect of the present invention provides a method for producing a fucoxanthin-containing composition. This method for producing a fucoxanthin-containing composition includes a step of culturing a diatom in a medium at pH 1.0 or more and pH 4.0 or less, wherein the diatom has 18S rRNA composed of the following (a) or (b): (a) a polynucleotide consisting of a nucleotide sequence represented by SEQ ID NO: 1; or (b) a polynucleotide consisting of a nucleotide sequence having 85% or more identity to the nucleotide sequence represented by SEQ ID NO: 1. The method for producing a fucoxanthin-containing composition according to this item can be used to produce a fucoxanthin-containing composition having improved antimicrobial properties because the fucoxanthin-containing composition is produced by culturing a diatom having 18S rRNA composed of the above (a) or (b) in a medium at pH 1.0 or more and pH 4.0 or less.
(2) In the method for producing a fucoxanthin-containing composition according to (1) above, the medium may contain silicon corresponding to sodium metasilicate nonahydrate at 100 mg/L or more and 10 g/L or less. According to the method for producing a fucoxanthin-containing composition according to this item, it is possible to suppress a shortage of silicic acid required for the synthesis of the silica frustules of diatoms. As a result, since the growth of diatom can be promoted, the production efficiency of the fucoxanthin-containing composition can be enhanced.
(3) In the method for producing a fucoxanthin-containing composition according to (1) or (2) above, the pH of the medium may be pH 1.5 or more and pH 2.5 or less. The method for producing a fucoxanthin-containing composition according to this item can be used to prevent a decrease in productivity during production of a fucoxanthin-containing composition having improved antimicrobial properties because the fucoxanthin-containing composition is produced by culturing a diatom having 18S rRNA composed of the above (a) or (b) in a medium at pH 1.5 or more and pH 2.5 or less.
(4) In the method for producing a fucoxanthin-containing composition according to any one of (1) to (3) above, the medium may contain seawater. The method for producing a fucoxanthin-containing composition according to this item can be used to prevent the growth of organisms that are difficult to grow in seawater because the fucoxanthin-containing composition is produced using a seawatercontaining medium. As a result, contamination during production can be suppressed, so that a decrease in productivity of the fucoxanthin-containing composition can be suppressed.
(5) In the method for producing a fucoxanthin-containing composition according to any one of (1) to (4) above, a sodium ion concentration of the medium may be 0.1 M or more and 0.6 M or less. The method for producing a fucoxanthin-containing composition according to this item can be used to prevent contamination during production because the fucoxanthin-containing composition is produced using a medium having a sodium ion concentration of 0.1 M or more and 0.6 M or less, and as a result, a decrease in productivity of the fucoxanthin-containing composition can be suppressed.
(6) In the method for producing a fucoxanthin-containing composition according to any one of (1) to (5) above, the medium may be a liquid medium, and the method may further include, after the culturing step, a sedimentation step of settling the diatom by allowing the liquid medium to stand; and a step of removing the liquid medium after the sedimentation step. The method for producing a fucoxanthin-containing composition according to this item can be used to prevent the fucoxanthin-containing composition production step from becoming complicated because the liquid medium is removed after the diatom is settled by allowing the liquid medium to stand.
(7) Another aspect of the present disclosure provides a fucoxanthin-containing composition. This fucoxanthin-containing composition includes a diatom and fucoxanthin, wherein the diatom has 18S rRNA composed of the following (a) or (b) and is viable at pH 1.0 or more and pH 4.0 or less: (a) a polynucleotide consisting of a nucleotide sequence represented by SEQ ID NO: 1; or (b) a polynucleotide consisting of a nucleotide sequence having 85% or more identity to the nucleotide sequence represented by SEQ ID NO: 1. The fucoxanthin-containing composition of this item has excellent antimicrobial properties.
(8) The fucoxanthin-containing composition according to (7) above may further include silicon. The fucoxanthin-containing composition of this item makes it possible to omit or simplify the step of removing silicon from the fucoxanthin-containing composition.
(9) In the fucoxanthin-containing composition according to (7) or (8) above, a ratio of a content of the fucoxanthin to a content of the diatom may be 0.1 mass% or more and 5 mass% or less in terms of dry mass. According to the fucoxanthin-containing composition of this item, a decrease in the stability of fucoxanthin can be prevented.
(10) In the fucoxanthin-containing composition according to (7) or (8) above, the ratio of the content of the fucoxanthin to the content of the diatom may be 50 mass% or more and 99.9 mass% or less in terms of dry mass. According to the fucoxanthin-containing composition of this item, highly concentrated fucoxanthin can be provided.
**(11)** In the fucoxanthin-containing composition according to any one of (7) to (10) above, the diatom may be viable at pH 1.5 or more and pH 2.5 or less. The fucoxanthin-containing composition of this item has better antimicrobial properties.
(12) Still another aspect of the present disclosure provides a food containing the fucoxanthin-containing composition according to any one of (7) to (11) above.
(13) Still another aspect of the present disclosure provides a cosmetic containing the fucoxanthin-containing composition according to any one of (7) to (11) above.
(14) Still another aspect of the present disclosure provides a diatom. This diatom includes 18S rRNA composed of the following (a) or (b) and is viable at pH 1.0 or more and pH 4.0 or less: (a) a polynucleotide consisting of a nucleotide sequence represented by SEQ ID NO: 1; or (b) a polynucleotide consisting of a nucleotide sequence having 85% or more identity to the nucleotide sequence represented by SEQ ID NO: 1. The diatom of this item can be used for producing a fucoxanthin-containing composition having improved antimicrobial properties.

Note that the present invention can be implemented in various forms, and examples thereof include: use of a diatom in the manufacture of a fucoxanthin-containing composition; a pharmaceutical product containing a fucoxanthin-containing composition; a quasi-drug product containing a fucoxanthin-containing composition; a skin external preparation containing a fucoxanthin-containing composition; a supplement containing a fucoxanthin-containing composition; a method for producing a food containing a fucoxanthin-containing composition; and a method for producing a cosmetic containing a fucoxanthin-containing composition.

### [Brief Description of Drawings]

[Fig. 1] Microscopic images showing diatoms used in Experiment 1.
[Fig. 2] A graph showing the results of a freshwater medium in Experiment 1.
[Fig. 3] A graph showing the results of a seawater medium in Experiment 1.
[Fig. 4] A graph showing the results of measuring a cell density.
[Fig. 5] A graph showing the results of measuring a chlorophyll concentration.
[Fig. 6] A graph showing a cell production amount.
[Fig. 7] Chromatograms showing the results of HPLC analysis.
[Fig. 8] A graph showing a fucoxanthin production rate.
[Fig. 9] A graph showing the results of measuring a cell density in Experiment 4.
[Fig. 10] A graph showing the results of measuring chlorophyll fluorescence in Experiment 4.
[Fig. 11] A graph showing the results of chlorophyll fluorescence/cell density in Experiment 4.
[Fig. 12] A graph showing the results of measuring the pH of culture medium in Experiment 4.

### [Description of Embodiments]

An embodiment of the present disclosure provides a method for producing a fucoxanthin-containing composition. This method for producing a fucoxanthin-containing composition includes a step of culturing a diatom in a medium at pH 1.0 or more and pH 4.0 or less. This diatom has 18S rRNA composed of the following (a) or (b):
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.

The medium used in the culturing step (hereinafter, also referred to as "culture step") is not particularly limited, and may be, for example, a CSi medium, an f/2 medium, or an IMK medium, may be prepared with freshwater, or may contain seawater. When the medium contains seawater, the growth of organisms that are difficult to grow in seawater can be suppressed. More specifically, it is possible to suppress the growth of organisms such as protozoa and microorganisms having no salt resistance, in addition to organisms having no acid resistance. As a result, contamination during culture can be suppressed, so that a decrease in productivity of the fucoxanthin-containing composition can be suppressed. In addition, since the medium can be prepared using seawater even in an environment where freshwater cannot be used, the fucoxanthin-containing composition can be produced in a wide range of environments. The seawater is not particularly limited, and examples thereof include collected seawater and artificial seawater. The sodium ion concentration of the medium is not particularly limited, but is preferably 0.05 M or more from the viewpoint of suppressing contamination during culture, and is preferably 1.0 M or less from the viewpoint of improving the production efficiency of the fucoxanthin-containing composition. The sodium ion concentration of the medium is more preferably 0.1 M or more and 0.6 M or less, and still more preferably 0.2 M or more and 0.5 M or less.

The medium preferably contains silicon from the viewpoint of promoting the growth of diatom to improve the production efficiency of the fucoxanthin-containing composition. The concentration of silicon contained in the medium is not particularly limited, but is preferably 10 mg/L or more in terms of sodium metasilicate nonahydrate from the viewpoint of suppressing a shortage of silicic acid required for the synthesis of the silica frustules of diatoms. The concentration of silicon contained in the medium is preferably 10 g/L or less in terms of sodium metasilicate nonahydrate from the viewpoint of suppressing the addition of silicic acid at a concentration more than necessary and the aggregated precipitation of the medium components. The concentration of silicon contained in the medium is preferably 10 mg/L or more and 8 g/L or less, more preferably 50 mg/L or more and 6 g/L or less, and still more preferably 100 mg/L or more and 4 g/L or less in terms of sodium metasilicate nonahydrate. The concentration of silicon contained in the medium may be 100 mg/L or more and 10 g/L or less in terms of sodium metasilicate nonahydrate. In addition, nitrogen, nutrient salts such as phosphates, vitamins, and trace elements may be added to the medium.

When the pH of the medium is 1.0 or more and 4.0 or less, the growth of microorganisms that are difficult to grow in an acidic environment can be suppressed, so that contamination during culture can be suppressed. The pH of the medium is preferably 3.5 or less, more preferably 3.0 or less, and still more preferably 2.5 or less from the viewpoint of efficiently suppressing the growth of microorganisms that are difficult to grow in an acidic environment. In addition, the pH of the medium is preferably 1.5 or more from the viewpoint of improving the production efficiency of the fucoxanthin-containing composition. Accordingly, the pH of the medium is preferably 1.5 or more and 3.5 or less, more preferably 1.5 or more and 3.0 or less, and still more preferably 1.5 or more and 2.5 or less. In particular, the sodium ion concentration of the medium may be 0.1 M or more and 0.6 M or less. In this case, for example, when the medium contains seawater, the pH of the medium is preferably 1.0 or more and 3.5 or less, more preferably 1.2 or more and 3.5 or less, still more preferably 1.5 or more and 3.5 or less, even more preferably 1.5 or more and 3.0 or less, and particularly preferably 1.5 or more and 2.5 or less. In addition, the concentration of sodium ions contained in the medium may be less than 0.1 M. In this case, the pH of the medium is preferably 1.5 or more and 3.5 or less, more preferably 1.5 or more and 3.0 or less, still more preferably 1.5 or more and 2.5 or less, and still even more preferably 2.0 or more and 2.5 or less.

The form of the medium is not particularly limited, and may be a liquid medium or a solid medium, but is preferably a liquid medium from the viewpoint of facilitating mass culture. In addition, the culturing procedure in the culture step is not particularly limited. For example, a nutrient source may be continuously or intermittently added to the medium during culture. In addition, the culture may be in the form of culture using a solid carrier device, or may be in the form of culturing twodimensionally or three-dimensionally while using a substrate for cell immobilization or the like. Note that the medium may be changed or replaced in the middle of the culture step.

The culture step is preferably performed by a procedure with enhanced air permeability, and for example, continuous or intermittent aeration culture, shaking culture, stirring culture, aeration stirring culture, and the like may be performed. Further, the culture step may be performed under a light irradiation condition, and for example, the culture may be continuously or periodically irradiated with white or green light. The culture temperature in the culture step is not particularly limited, and may be, for example, 10°C to 30°C. In addition, the culture period in the culture step is not particularly limited, but may be, for example, one week to eight weeks. In addition, a culture place in the culture step is not particularly limited, but may be, for example, a greenhouse, outdoor, or indoor.

In the culture step, a diatom having 18S rRNA composed of the above (a) or (b) is cultured. The identity of the nucleotide sequence described in the above (b) means the maximum identity (%) between nucleotide sequences obtained by aligning (alignment) two nucleotide sequences to be compared while introducing gaps, as necessary. The identity of the nucleotide sequence can be calculated, for example, by blastn of NCBI BLAST (http://blast.ncbi.nlm.nih.gov/) where the BLAST algorithm is implemented, Homolog search implemented in Genetyx Win, or the like.

From the viewpoint of enhancing the production efficiency of the fucoxanthin-containing composition, the diatom preferably has, as 18S rRNA, a polynucleotide consisting of a nucleotide sequence having 90% or more identity to the nucleotide sequence represented by SEQ ID NO: 1, more preferably has a polynucleotide consisting of a nucleotide sequence having 95% or more identity to the nucleotide sequence represented by SEQ ID NO: 1, and still more preferably has a polynucleotide consisting of a nucleotide sequence having 97% or more identity to the nucleotide sequence represented by SEQ ID NO: 1.

The diatom having 18S rRNA composed of the above (a) or (b) is not particularly limited, but is preferably a pennate diatom. The pennate diatom is not particularly limited, but is preferably a diatom belonging to the genus *Pinnularia.* The diatom having 18S rRNA composed of the above (a) or (b) is not particularly limited, and may be, for example, a diatom, the accession number of which is FERM P-22467.

The amount of cells increased in the culture step, that is, the growth rate is not particularly limited, but is preferably 0.5 mg/L/day or more, more preferably 5 mg/L/day or more, and still more preferably 50 mg/L/day or more in terms of dry weight. The amount of fucoxanthin increased in the culture step, that is, the production rate of fucoxanthin is not particularly limited, but is preferably 0.002 mg/L/day or more, more preferably 0.02 mg/L/day or more, and still more preferably 0.2 mg/L/day or more in terms of dry weight. The amount of fucoxanthin increased in the culture step can be determined by comparison with the growth rate of diatom.

The method for producing a fucoxanthin-containing composition may further include a step of removing the medium after the culture step. The step of removing the medium is not particularly limited, and for example, when the medium is a liquid medium, the step may include a step of settling diatom cells by allowing the liquid medium to stand, a step of settling diatom cells by centrifuging the liquid medium, and the like. The step of removing the medium preferably includes a sedimentation step of settling the diatom by allowing the liquid medium to stand and a step of removing the liquid medium after the sedimentation step, from the viewpoint of simplifying the step of recovering the diatom cells. This method can suppress complication of the step of producing the fucoxanthin-containing composition, and it is thus possible to suppress an increase in the production cost of the fucoxanthin-containing composition.

The method for producing a fucoxanthin-containing composition may further include, after the culture step, an extraction step of extracting fucoxanthin and a purification step of purifying the fucoxanthin. The extraction step and the purification step can be performed according to a known procedure. The extraction step is not particularly limited, and examples thereof include: a process in which a culture medium is allowed to stand, and diatom cells are precipitated, recovered, and then extracted with a solvent such as an organic solvent; a process in which diatom cells are recovered on a filter such as glass fiber, dried, and then extracted with a solvent such as an organic solvent; and a process in which cells are crushed by a means such as a French press or a homogenizer, and then extracted with a solvent. The solvent for extracting fucoxanthin from the diatom is not particularly limited as long as fucoxanthin can be extracted therewith, and examples thereof include alcohols such as methanol, ethanol, propanol, isopropanol, and n-butanol, ketones such as methyl ethyl ketone and acetone, esters such as methyl acetate and ethyl acetate, organic chlorinated hydrocarbons such as chloroform, aliphatic hydrocarbons such as hexane, aromatic hydrocarbons such as benzene and toluene, and acetone. These solvents can be used singly or in combination of two or more kinds thereof. The purification step is not particularly limited, but for example, the solvent extract obtained in the extraction step as it is or after the residue is removed may be subjected to reverse phase chromatography or the like using an open column, a flash column, or a high performance liquid chromatograph.

The method for producing a fucoxanthin-containing composition according to the present embodiment as described above can be used to produce a fucoxanthin-containing composition having improved antimicrobial properties because the fucoxanthin-containing composition is produced by culturing a diatom having 18S rRNA composed of the above (a) or (b) in a medium at pH 1.0 or more and pH 4.0 or less. In addition, since the fucoxanthin-containing composition is produced by culturing a diatom in a medium at pH 1.0 or more and pH 4.0 or less, it is possible to suppress the growth of organisms having no acid resistance, and fucoxanthin can be produced under conditions in which contamination is suppressed. For this reason, for example, it is possible to suppress contamination of protozoa and the like that prey on diatoms, bacteria and fungi in which nutrient salts compete with diatoms, and the like. It is thus possible to suppress a decrease in productivity of the fucoxanthin-containing composition. In addition, as a result of being able to suppress contamination, culture can be performed using a simple culture apparatus such as open culture, so that an increase in the production cost of the fucoxanthin-containing composition can be suppressed. In addition, since the fucoxanthin-containing composition is produced by causing the diatom to produce fucoxanthin, it is possible to suppress a decrease in productivity of the fucoxanthin-containing composition.

The second embodiment of the present disclosure provides a fucoxanthin-containing composition including a diatom and fucoxanthin. In this fucoxanthin-containing composition, the diatom has 18S rRNA composed of (a) or (b) below and is viable at pH 1.0 or more and pH 4.0 or less:
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.

From the viewpoint of enhancing the antimicrobial properties of the fucoxanthin-containing composition, the diatom is preferably viable at pH 1.0 or more and pH 3.0 or less, and more preferably viable at pH 1.5 or more and pH 2.5 or less. The diatom preferably has, as 18S rRNA, a polynucleotide consisting of a nucleotide sequence having 90% or more identity to the nucleotide sequence represented by SEQ ID NO: 1, more preferably has a polynucleotide consisting of a nucleotide sequence having 95% or more identity to the nucleotide sequence represented by SEQ ID NO: 1, and still more preferably has a polynucleotide consisting of a nucleotide sequence having 97% or more identity to the nucleotide sequence represented by SEQ ID NO: 1. The diatom having 18S rRNA composed of the above (a) or (b) is not particularly limited, but is preferably a pennate diatom. The pennate diatom is not particularly limited, but is preferably a diatom belonging to the genus *Pinnularia.* The diatom having 18S rRNA composed of the above (a) or (b) is not particularly limited, and may be, for example, a diatom, the accession number of which is FERM P-22467.

The fucoxanthin-containing composition including a diatom and fucoxanthin may be obtained, for example, by recovering cells from a medium after the culture step in the method for producing a fucoxanthin-containing composition as described above. The process of recovering cells is not particularly limited, and examples thereof include: a process in which diatom cells are settled by allowing a liquid medium to stand and then recovered; a process in which cells are recovered by filtering a culture medium with a mesh filter; and a process in which cells are recovered using a continuous centrifuge equipped with a nozzle type separator. These processes may each be used to recover the slurry-like cells

The fucoxanthin-containing composition may be in a liquid state, a solid state, or a dry state such as powder. The method for obtaining a fucoxanthin-containing composition in a dry state is not particularly limited, and examples thereof include a method in which cells recovered by the above process are dried using a spray dryer, a freeze dryer, a vacuum dryer, or the like.

In the fucoxanthin-containing composition, the ratio of the content of fucoxanthin to the content of diatom is not particularly limited, but is preferably 0.01 mass% or more, more preferably 0.1 mass% or more, still more preferably 0.5 mass% or more, and even still more preferably 1.0 mass% or more in terms of dry mass from the viewpoint of suppressing a decrease in stability of fucoxanthin. In addition, the ratio is preferably 10 mass% or less, more preferably 7 mass% or less, and still more preferably 5 mass% or less in terms of dry mass from the viewpoint of suppressing a decrease in stability of fucoxanthin. In view of the above, the ratio of the content of fucoxanthin to the content of diatom may be 0.1 mass% or more and 5 mass% or less in terms of dry mass, for example.

In the fucoxanthin-containing composition, from the viewpoint of suppressing decomposition of fucoxanthin, it is preferable that the diatom cells that have produced fucoxanthin are directly used as dry powder in an embodiment. According to such an embodiment, as compared with an embodiment in which fucoxanthin is extracted, fucoxanthin can be stabilized, so that decomposition of fucoxanthin can be suppressed. For example, as compared with an embodiment in which fucoxanthin is extracted with a solvent such as ethanol and pulverized, decomposition of fucoxanthin can be suppressed over a long period of time. In addition, as compared with an embodiment in which fucoxanthin is extracted with a solvent such as ethanol, powdered, and then dissolved in a solvent such as ethanol, decomposition of fucoxanthin can be further suppressed over a long period of time.

In the fucoxanthin-containing composition, the concentration of fucoxanthin may be increased through the extraction step as described above, and the concentration of fucoxanthin may be further increased through the purification step as described above. Incidentally, the amount of diatom contained in the fucoxanthin-containing composition subjected to the extraction step and the purification step should be very small. However, the diatom can be detected using a highly sensitive analysis method such as PCR using the above-described 18S rRNA, that is, a primer designed based on the nucleotide sequence represented by SEQ ID NO: 1, or time-of-flight mass spectrometry.

In the fucoxanthin-containing composition in an embodiment in which the concentration of fucoxanthin is increased by an extraction step or the like, the ratio of the content of fucoxanthin to the content of diatom is not particularly limited. From the viewpoint of providing highly concentrated fucoxanthin, the ratio is preferably 50 mass% or more, more preferably 60 mass% or more, still more preferably 70 mass% or more, even still more preferably 80 mass% or more, further still more preferably 90 mass% or more, particularly preferably 95 mass% or more, and further particularly preferably 97 mass% or more in terms of dry mass. In addition, from the viewpoint of productivity, the ratio is preferably 99.9 mass% or less, more preferably 99.8 mass% or less, and still more preferably 99.5 mass% or less in terms of dry mass. In view of the above, the ratio of the content of fucoxanthin to the content of diatom may be 50 mass% or more and 99.9 mass% or less in terms of dry mass.

The fucoxanthin-containing composition may further include silicon. In other words, the fucoxanthin-containing composition may contain a component derived from diatom frustules. According to an embodiment in which the fucoxanthin-containing composition contains silicon, it is possible to omit or simplify a step of removing silicon, that is, a component derived from diatom frustules, from the fucoxanthin-containing composition. As a result, it is possible to suppress complication of the step of producing the fucoxanthin-containing composition, so that an increase in production cost can be suppressed. The step of removing a component derived from diatom frustules is not particularly limited, and examples thereof include a step of extracting fucoxanthin by using a solvent. The concentration of silicon contained in the fucoxanthin-containing composition is not particularly limited, but is preferably 20 mass% or more and 80 mass% or less, and more preferably 50 mass% or more and 70 mass% or less. The concentration of silicon contained in the fucoxanthin-containing composition can be determined according to the method described in J. Grad. Sch. Biosp. Sci., Hiroshima Univ. (2006), 45: 21-29 while using the molybdenum blue method. Note that the fucoxanthin-containing composition may contain other optional components, and for example, may contain an additive such as dextran, a stabilizer such as an antioxidant, or the like.

The fucoxanthin-containing composition of the present embodiment may be used as a raw material for various foods and beverages. The form of the raw material for a food and beverage is not particularly limited, and examples thereof include powder, granules, pills, soft capsules, hard capsules, tablets, and liquids.

The third embodiment of the present disclosure provides a food including the fucoxanthin-containing composition. As food products, there are no particular limitations, but examples thereof include: breads such as white bread and butter rolls; confectioneries such as baked sweets and cakes; noodles such as udon and soba; ricebased foods such as rice balls and rice porridge; mochi products such as kusa-mochi and kinako-mochi; pasta products such as spaghetti, fettuccine, penne, eliche, ravioli, and lasagna; dressings such as mayonnaise, salad creamy dressings, semi-solid dressings, emulsified liquid dressings, and separated liquid dressings; and foods for the sick or elderly such as mousses, jellies, and soups. In addition, the food containing the fucoxanthin-containing composition may also be a so-called health food, such as a supplement, food for specified health use, nutritional supplement, or nutrient preparation.

In the food product containing the fucoxanthin-containing composition, the fucoxanthin-containing composition is preferably in the form of dry powder of cells recovered by the process as described above. The amount of the fucoxanthin-containing composition contained in the food is not particularly limited, and may be appropriately set according to the food. The amount of the fucoxanthin-containing composition contained in the food is preferably 0.1 mass% or more and 98 mass% or less, more preferably 0.5 mass% or more and 95 mass% or less, and still more preferably 1 mass% or more and 90 mass% or less. For example, when the food is pasta, the amount is preferably 0.5 mass% or more and 20 mass% or less, and more preferably 1 mass% or more and 10 mass% or less. In addition, for example, when the food is a supplement, the amount is preferably 50 mass% or more and 98 mass% or less, and more preferably 70 mass% or more and 95% or less. In applications in which the fucoxanthin concentration is preferably high, such as supplements and health foods, the fucoxanthin-containing composition may have a form in which the fucoxanthin concentration is increased by the extraction step or the like as described above. For example, when the food is a health food, the fucoxanthin concentration in the health food is preferably 10 mass% or more and 98 mass% or less, and more preferably 20 mass% or more and 95% or less.

In addition, the fucoxanthin-containing composition of the present disclosure may be used as a raw material for cosmetics.

The fourth embodiment of the present disclosure provides a cosmetic including the fucoxanthin-containing composition. The cosmetic is not particularly limited, but may be for the purpose of, for example, preventing dullness of the skin, preventing roughness of the skin, preventing spots, improving wrinkles, rejuvenating the skin, and preventing acne. Meanwhile, the form of the cosmetic is not particularly limited, and examples thereof include emulsions, creams, lotions, beauty serums, facial masks, cleansers, makeup cosmetics such as foundations, blushes, and lipsticks, scalp care products such as hair growth tonics, hair tonics, shampoos, and conditioners, dispersions, ointments, solutions, aerosols, and patches.

In the cosmetic containing the fucoxanthin-containing composition, the fucoxanthin-containing composition may be in the form of dry powder of cells recovered by the process as described above, but is preferably in the form in which the fucoxanthin concentration is further increased by the extraction step or the like as described above. The amount of the fucoxanthin-containing composition contained in the food is not particularly limited, and may be appropriately set according to the cosmetic. The amount of the fucoxanthin-containing composition contained in the cosmetic is preferably 0.001 mass% or more and 10 mass% or less, more preferably 0.005 mass% or more and 5 mass% or less, and still more preferably 0.01 mass% or more and 2 mass% or less. For example, when the cosmetic is a beauty serum, the fucoxanthin concentration in the beauty serum is preferably 0.001 mass% or more and 1 mass% or less, and more preferably 0.005 mass% or more and 0.5 mass% or less.

In addition, the fucoxanthin-containing composition of the present disclosure may be used as a raw material for pharmaceutical products or quasi-pharmaceutical products. The use is not particularly limited, but examples thereof include antiinflammatory agents, angiogenesis inhibitors, therapeutics for virus-related malignant tumors, antioxidants and cell-activating agents, triglyceride absorption regulators, antiobesity agents, treatments and preventives for lipid metabolism disorders, antitumor agents, cancer cell proliferation inhibitors, blood glucose level suppressants, topical agents for the scalp, antidiabetic drugs, preventives and treatments for hyperlipidemia, topical dermatological agents, neuroprotective agents, and veterinary drugs. As the form, various forms such as pills, powders, and capsules can be selected, as necessary. In addition, the fucoxanthin-containing composition of the present disclosure can be used for various applications such as research reagents. In the pharmaceutical product or the research reagent containing the fucoxanthin-containing composition, the fucoxanthin-containing composition is preferably in a form in which the fucoxanthin concentration is increased by the extraction step or purification step as described above. For example, in the case of use in a research reagent, the fucoxanthin concentration in the research reagent is preferably 90 mass% or more and 99.9 mass% or less, and more preferably 95 mass% or more and 99.5 mass% or less.

The solution in which the fucoxanthin-containing composition of the present disclosure is dissolved should be acidic. Thus, the fucoxanthin-containing composition of the present disclosure has excellent antimicrobial properties. As a result, the antimicrobial properties can be improved in the above-mentioned various applications such as foods and cosmetics containing the fucoxanthin-containing composition.

The fifth embodiment of the present disclosure provides a diatom. This diatom has 18S rRNA composed of (a) or (b) below and is viable at pH 1.0 or more and pH 4.0 or less:
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.

The diatom of this embodiment allows for production of a fucoxanthin-containing composition having improved antimicrobial properties. From the viewpoint of enhancing the antimicrobial properties of the fucoxanthin-containing composition, the diatom is preferably viable at pH 1.0 or more and pH 3.0 or less, and more preferably viable at pH 1.5 or more and pH 2.5 or less. The diatom preferably has, as 18S rRNA, a polynucleotide consisting of a nucleotide sequence having 90% or more identity to the nucleotide sequence represented by SEQ ID NO: 1, more preferably has a polynucleotide consisting of a nucleotide sequence having 95% or more identity to the nucleotide sequence represented by SEQ ID NO: 1, and still more preferably has a polynucleotide consisting of a nucleotide sequence having 97% or more identity to the nucleotide sequence represented by SEQ ID NO: 1. The diatom having 18S rRNA composed of the above (a) or (b) is not particularly limited, but is preferably a pennate diatom. The pennate diatom is not particularly limited, but is preferably a diatom belonging to the genus *Pinnularia.* The diatom having 18S rRNA composed of the above (a) or (b) is not particularly limited, and may be, for example, a diatom, the accession number of which is FERM P-22467.

### [Examples]

Hereinafter, the present invention will be described more specifically with reference to Examples. The present invention, however, is not limited to the following Examples.

### 1. Experiment 1 (Experiment on growth of diatom depending on pH)

### (1) Procedure

For the diatom having 18S rRNA as described above, the pH at which the diatom could grow was examined. As the diatom, a diatom strain indicated by accession number FERM P-22467 was used. The medium used was a freshwater medium having the composition shown in Table 1 below or a seawater medium having the composition shown in Table 2 below. The pH of each medium was adjusted to 1.5, 2.0, 3.0, 4.0, 5.0, and 6.0. In the seawater medium, a medium at pH 1.0, pH 1.1, pH 1.2, pH 1.3, or pH 1.4 was also used. Then, 3 mL of the medium was dispensed into a 24-well plate. Static culture was performed for 10 days at a culture temperature of 26°C under a light intensity of 10 µE using a white fluorescent lamp. Using a fluorometer Qubit3 Fluorometer (manufactured by ThermoFisher Scientific, Inc.), chlorophyll fluorescence of the culture medium at 665 to 720 nm was measured using blue light excitation at 430 to 495 nm and a red fluorescence filter, and the growth rate of cells was quantified.

### [Table 1]

**Table 1**

| Component | Content | |
|---|---|---|
| NaNO₃ | 0.68 | g/L |
| KH₂PO₄ | 0.136 | g/L |
| Na₂SiO₃•9H₂O | 2.273 | g/L |
| MgSO₄•7H₂O | 0.075 | g/L |
| CaCl₂•2H₂O | 0.036 | g/L |
| EDTA-Fe | 44 | mg/L |
| H₃BO₃ | 5.7 | mg/L |
| FeCl₃•6H₂O | 6.32 | mg/L |
| MnCl₂•4H₂O | 3.6 | mg/L |
| ZnCl₂ | 0.21 | mg/L |
| Na₂MoO₄•2H₂O | 0.78 | mg/L |
| CoCl₂•6H₂O | 0.08 | mg/L |
| CuCl₂•2H₂O | 0.086 | mg/L |
| Na₂SeO₃ | 0.173 | mg/L |
| H₂SO₄ | After each component was added, added to adjust a pH to 2.5. | |

### [Table 2]

**Table 2**

| Component | Content | |
|---|---|---|
| NaNO₃ | 0.68 | g/L |
| KH₂PO₄ | 0.136 | g/L |
| Na₂SiO₃•9H₂O | 2.273 | g/L |
| MgSO₄•7H₂O | 5.5 | g/L |
| CaCl₂•2H₂O | 0.5g | g/L |
| KCl | 0.5 | g/L |
| NaCl | 25 | g/L |
| EDTA-Fe | 44 | mg/L |
| H₃BO₃ | 5.7 | mg/L |
| FeCl₃•6H₂O | 6.32 | mg/L |
| MnCl₂•4H₂O | 3.6 | mg/L |
| ZnCl₂ | 0.21 | mg/L |
| Na₂MoO₄•2H₂O | 0.78 | mg/L |
| CoCl₂•6H₂O | 0.08 | mg/L |
| CuCl₂•2H₂O | 0.086 | mg/L |
| Na₂SeO₃ | 0.173 | mg/L |
| H₂SO₄ | After each component was added, added to adjust a pH to 2.5. | |

### (2) Results and Discussion

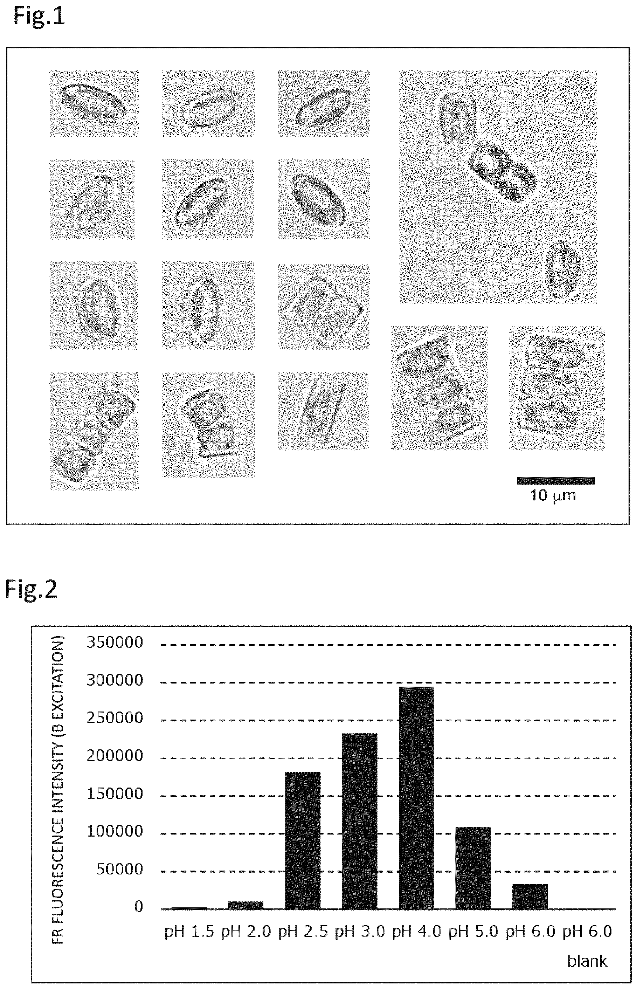

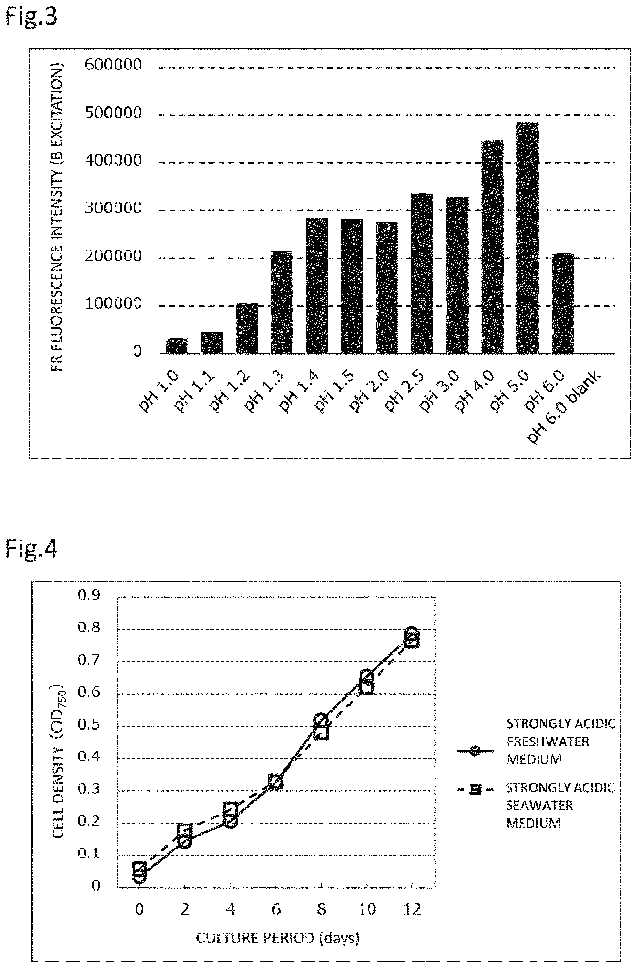

Fig. 1 is microscopic images showing diatoms used in Experiment 1. Fig. 2 is a graph showing the results of a freshwater medium in Experiment 1. Fig. 3 is a graph showing the results of a seawater medium in Experiment 1. In Figs. 2 and 3, the abscissa represents pH, and the ordinate represents the intensity (B excitation) of chlorophyll fluorescence. From the results shown in Fig. 2, it has been demonstrated that in the freshwater medium, the diatom used in Experiment 1 can grow at least in the range of pH 1.5 to pH 6.0 where the experiment was performed. Therefore, it has been suggested that the diatom is viable at least in the range of pH 1.5 to pH 6.0. From the results shown in Fig. 3, it has been demonstrated that in the seawater medium, the diatom used in Experiment 1 can grow at least in the range of pH 1.0 to pH 6.0 where the experiment was performed. Therefore, it has been suggested that the diatom is viable at least in the range of pH 1.0 to pH 6.0.

### 2. Experiment 2 (Experiment on production of fucoxanthin-containing composition)

### (1) Procedure

### <To culture and check cell density and chlorophyll concentration>

Regarding the diatom having 18S rRNA as described above, a fucoxanthin-containing composition was produced using a freshwater medium or a seawater medium. As the diatom, a diatom strain indicated by accession number FERM P-22467 was used like Experiment 1. As the medium, in the same manner as in Experiment 1, a strongly acidic freshwater medium (pH 2.5) having the composition shown in Table 1 above and a strongly acidic seawater medium (pH 2.5) having the composition shown in Table 2 above were used. Note that both media contain 2.273 g/L sodium metasilicate (Na₂SiO₃·9H₂O). The diatom strain was planted in 60 mL of the strongly acidic seawater medium contained in a 100-mL baffled flask, and was shake-cultured at a speed of 150 rpm for 12 days at a culture temperature of 26°C under a light intensity of 40 µE using a white fluorescent lamp. During the culture period, the cell density and chlorophyll concentration in the culture medium were checked every two days. The cell density was determined based on scattering at a wavelength of 750 nm in the culture medium while using a spectrophotometer V-650 (manufactured by JASCO Corporation). The chlorophyll concentration was determined by measuring fluorescence (665 to 495 nm) mainly derived from chlorophyll excited by blue light (430-720 nm) while using Qubit3 Fluorometer (manufactured by ThermoFisher Scientific, Inc.).

### <To recover cells>

The cultured cells were centrifuged at 5,000 g for 5 minutes at room temperature, and the cells were recovered. At the time of cell recovery, the volume (mL) of the culture medium was also checked. The recovered cells were lyophilized using a lyophilizer VD-800R (manufactured by Tomy Seiko Co., Ltd.), and the dry weight of the cells was measured.

### <To extract fucoxanthin>

To 20 mg of the dried and powdered cells, 1.0 mL of acetone was added, and the mixture was vigorously shaken at 30 Hz for 2 minutes by using a TissueLyser II homogenizer (manufactured by Qiagen). The lysate was centrifuged under conditions at room temperature for 10 minutes at 21,600 g, and the supernatant was collected. The supernatant was filtered through a 0.45-µm filter to obtain an extract.

### <To analyze fucoxanthin>

The extract was analyzed using a high-performance liquid chromatograph mass spectrometer Alliance HPLC/2998PDA/qDa system (manufactured by Waters) under the following analysis conditions. As a fucoxanthin standard, 95% grade fucoxanthin (manufactured by FUJIFILM Wako Pure Chemical Corporation) was diluted to 100 µg/mL, 50 µg/mL, and 10 µg/mL and used. A peak at 445 nm, which is a maximum absorption of fucoxanthin, was confirmed in a chromatogram.

| | |
|---|---|
| Injection sample volume: | 10 µL |
| Column used: | XBridge C18 3.5 µm, 4.6 mm × 150 mm |
| Column temperature: | 35°C |
| Flow rate: | 1mL/min |

### Solvent composition

| | |
|---|---|
| Solvent A: | 80% methanol, 19.9% water, 0.1% formic acid |
| Solvent B: | 99.9% methanol, 0.1% formic acid |
| Solvent C: | 100% acetonitrile |

### Gradient

0 to 10min: solvent A 100% to solvent B 100%
10 min to 20min: solvent B 100%
20 min to 30min: solvent C 100%

### (2) Results and Discussion

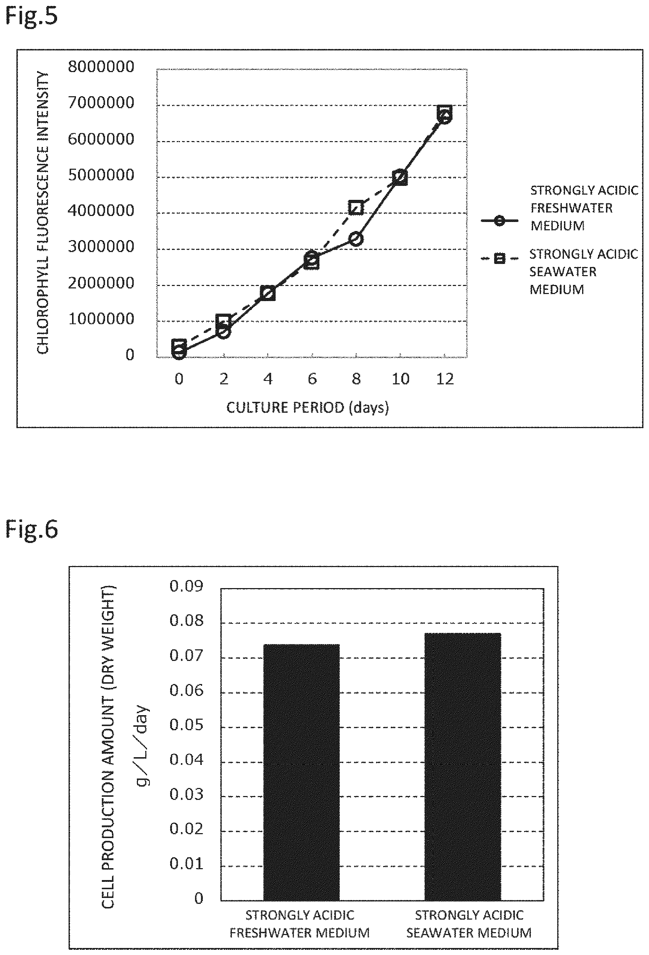

Fig. 4 is a graph showing the results of measuring a cell density. In Fig. 4, the abscissa represents the culture period (days), and the ordinate represents the cell density (OD₇₅₀). Fig. 5 is a graph showing the results of measuring a chlorophyll concentration. In Fig. 5, the abscissa represents the culture period (days), and the ordinate represents the chlorophyll fluorescence intensity. As shown in Fig. 4, the cell density linearly increased over 12 days of culture under both culture conditions using the strongly acidic freshwater medium and the strongly acidic seawater medium. In addition, as shown in Fig. 5, the chlorophyll concentration also increased linearly over 12 days of culture. These results indicate that the FERM AP-22467 strain can grow well in both the strongly acidic freshwater medium and the strongly acidic seawater medium.

Fig. 6 is a graph showing a cell production amount. In Fig. 6, the ordinate represents the cell production amount (g/L/day). The cell production amount is calculated from the volume (mL) of the culture medium at the time of cell recovery, the weight (mg) of the dried and powdered cells, and the number of days of culture. Incidentally, the cell production amount corresponds to the growth rate of cells. The cell production amount was found to be 0.074 g/L/day in the strongly acidic freshwater medium and 0.077 g/L/day in the strongly acidic seawater medium.

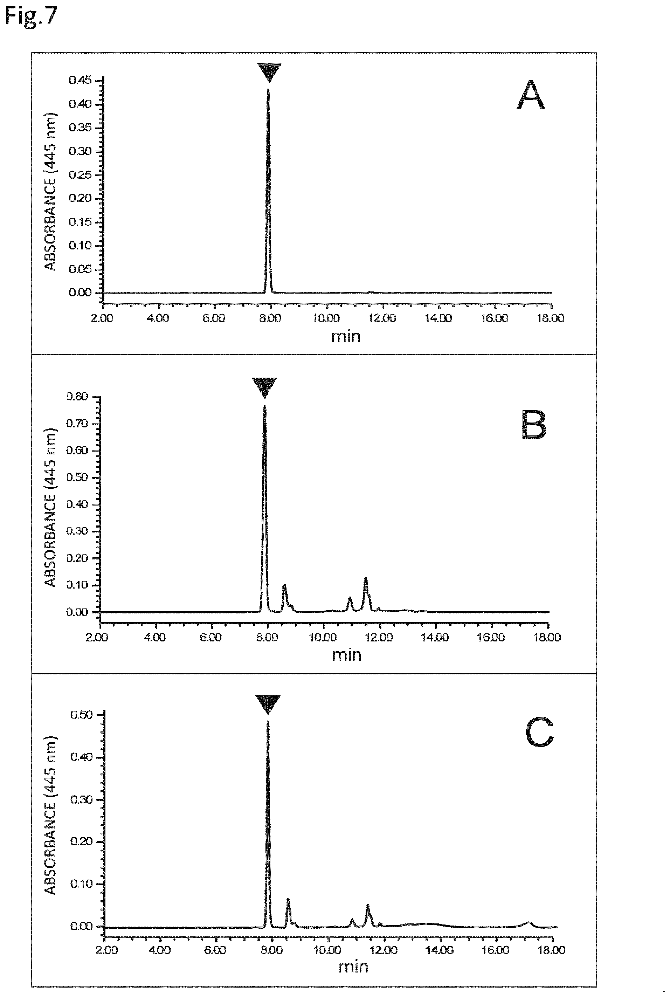

Fig. 7 is chromatograms showing the results of HPLC analysis. A shows the result of HPLC analysis of 50 µg/mL fucoxanthin standard, B shows the result of HPLC analysis of an extract of cells cultured in the strongly acidic freshwater medium, and C shows the result of HPLC analysis of an extract of cells cultured in the strongly acidic seawater medium. In both the case of culture using the strongly acidic freshwater medium and the case of culture using the strongly acidic seawater medium, a clear fucoxanthin peak was observed at the same retention time as that of the fucoxanthin standard. From the comparison with the peak obtained by the calibration curve, it was found that the concentration of fucoxanthin contained in the cells was 4.7 mg/g (0.47% (w/w)) in terms of dry weight under the strongly acidic freshwater medium culture condition, and 2.5 mg/g (0.25% (w/w)) under the strongly acidic seawater medium culture condition.

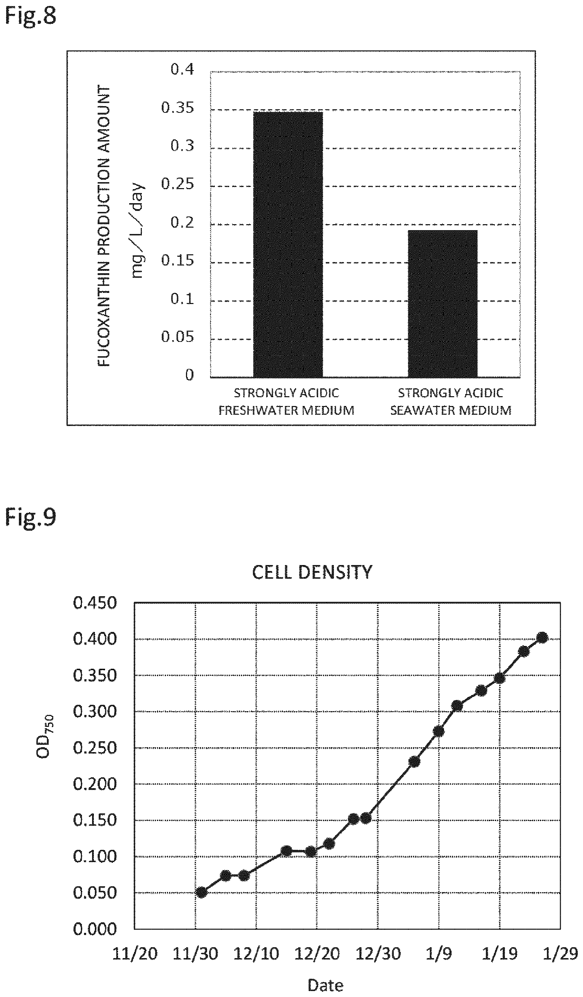

Fig. 8 is a graph showing a fucoxanthin production rate. In Fig. 8, the ordinate represents the fucoxanthin production rate (g/L/day). The fucoxanthin production rate is determined from the growth rate of cells and the concentration of fucoxanthin contained in the cells. The fucoxanthin production rate was found to be 0.35 mg/L/day in the strongly acidic freshwater medium and 0.19 mg/L/day in the strongly acidic seawater medium.

### 3. Experiment 3 (Experiment on antimicrobial properties)

### (1) Procedure

First, 0.5 g of the lyophilized powder of cells obtained under the culture conditions using the strongly acidic seawater medium in Experiment 2 was admixed well with 0.75 mL of water to form a paste. The pH of the surface of the paste was measured with a flat pH composite electrode 6261-10 C (manufactured by HORIBA, Ltd.). Further, the above paste was admixed well with 0.75 mL of water to form a slurry, and the pH was measured using the same electrode.

### (2) Results and Discussion

The paste-like cells had a pH of 4.59 and the slurry-like cells had a pH of 4.67. This revealed that the pH was maintained on the acidic side even when a certain amount of water was added to the lyophilized powder of the diatom strain indicated by accession number FERM P-22467 as cultured under strongly acidic conditions. It is generally known that acidic conditions inhibit the growth of unwanted microorganisms. This has indicated that the present lyophilized powder also has an action of suppressing contamination. Therefore, it has been suggested that the present lyophilized powder, that is, the fucoxanthin-containing composition in the present Example has excellent antimicrobial properties.

### 4. Experiment 4 (Experiment on scale-up)

### (1) Procedure

A 100-L water tank (sunlight tank SLP-100) was installed in a plastic greenhouse, and a diatom was cultured using about 90 L of freshwater medium while bubbling with an air pump. A plate of 30 cm × 50 cm was placed on the upper opening portion of the water tank, but the plate did not completely cover the opening portion, and the environment was such that insects and the like could easily enter. The air pump was not provided with a filter or the like for the purpose of preventing contamination, and was ventilated with unsterilized air. As the diatom, a diatom strain indicated by accession number FERM P-22467 was used. As the medium, a medium prepared by adding MgSO₄·7H₂O (0.5 g/L) to a freshwater medium having the composition shown in Table 1 above was used. The culture using the culture medium was started on November 17, and was carried out over the period shown in Table 3 below, and the pH of the culture medium was adjusted to 2.7 by adding dilute sulfuric acid on November 29 during the culture period. During the culture period, the chlorophyll fluorescence of the culture medium, the cell density of the culture medium, the pH of the culture medium, and the temperature of the culture medium were measured. The chlorophyll fluorescence of the culture medium was determined by measuring, in the culture medium diluted 10-fold with the medium, fluorescence (665 to 720 nm) excited by blue light (430 to 495 nm) while using a fluorometer Qubit3 Fluorometer (manufactured by ThermoFisher Scientific, Inc.). The cell density of the culture medium was determined based on scattering at a wavelength of 750 nm in the culture medium while using a spectrophotometer V-650 (manufactured by JASCO Corporation). The pH of the culture medium was measured using a pen-type pH meter (pH-222, manufactured by MOTHERTOOL CO., LTD.). Note that in Table 3, the time when the temperature of the culture medium was measured is also described.

### [Table 3]

**Table 3**

| Date | Qubit (x10) | OD750 | Qubit/OD750 | pH | Remarks (Temperature and temperature measurement time) |
|---|---|---|---|---|---|
| November 21 | 7617 | 0.019 | 400,895 | 3.08 | 9:45 10.8°C, 12:00 10.8°C, 14:00 12.6°C, 15:00 13.9°C |
| November 24 | 20090 | 0.026 | 772,692 | 3.11 | 9:45 17.4°C, 12:00 21.4°C |
| November 28 | 29714 | 0.046 | 645,957 | 3.17 | 11:00 17.4°C, 13:00 17.6°C, 15:00 17.6°C |
| November 29 | | | | | Adjust pH to 2.7 by using dilute sulfuric acid |
| December 1 | 36691 | 0.051 | 719,431 | 2.63 | 9:45 11.9°C, 13:00 14.8°C |
| December 5 | 53286 | 0.074 | 720,081 | 2.66 | 9:45 9.1°C 14:30 9.1°C |
| December 8 | 57641 | 0.074 | 778,932 | 2.67 | 10:00 12.4°C, 14:45 19.0°C |
| December 15 | 78385 | 0.108 | 725,787 | 2.67 | 9:45 13.6°C 11:00 13.7°C 14:30 13.7°C |
| December 19 | 86783 | 0.107 | 811,056 | 2.67 | 10:00 10.6°C, 11:30 13.0°C, 13:30 14.8°C |
| December 22 | 87521 | 0.118 | 741,703 | 2.86 | 9:45 4.7°C, 11:30 6.4°C, 13:30 8.1°C, 15:00 6.9°C |
| December 26 | 107824 | 0.152 | 709,368 | 2.72 | 9:45 9.3°C 11:15 12.3°C 13:00 13.5°C 15:00 14.6°C |
| December 28 | 131946 | 0.153 | 862,392 | 2.79 | 9:45 8.4°C, 11:00 9.4°C, 13:00 11.5°C, 15:00 14.3°C |
| January 5 | 203789 | 0.231 | 882,203 | 2.87 | 9:45 10.8°C, 11:15 12.9°C, 13:00 15.2°C, 15:00 17.1°C |
| January 9 | 223868 | 0.273 | 820,029 | 2.88 | 9:45 4.8°C, 11:00 8.3°C, 13:00 10.5°C, 15:00 11.9°C |
| January 12 | 221957 | 0.308 | 720,640 | 2.82 | 9:45 6.0°C 11:30 8.9°C 13:00 10.9°C 15:00 12.6°C |
| January 16 | 262140 | 0.329 | 796,778 | 2.84 | 9:45 5.5°C, 11:30 6.2°C, 13:00 7.6°C, 15:00 10.5°C |
| January 19 | 263214 | 0.346 | 760,734 | 2.88 | 9:45 7.8°C, 11:30 9.8°C, 13:00 11.4°C, 15:00 14.1°C |
| January 23 | 287907 | 0.383 | 751,715 | 2.88 | 9:45 9.0°C, 11:00 9.5°C, 13:00 12.0°C, 15:00 12.0°C |
| January 26 | 324984 | 0.402 | 808,418 | 2.94 | 9:45 4.2°C, 11:00 4.1°C, 13:00 7.9°C, 15:00 9.3°C |

### (2) Results and Discussion

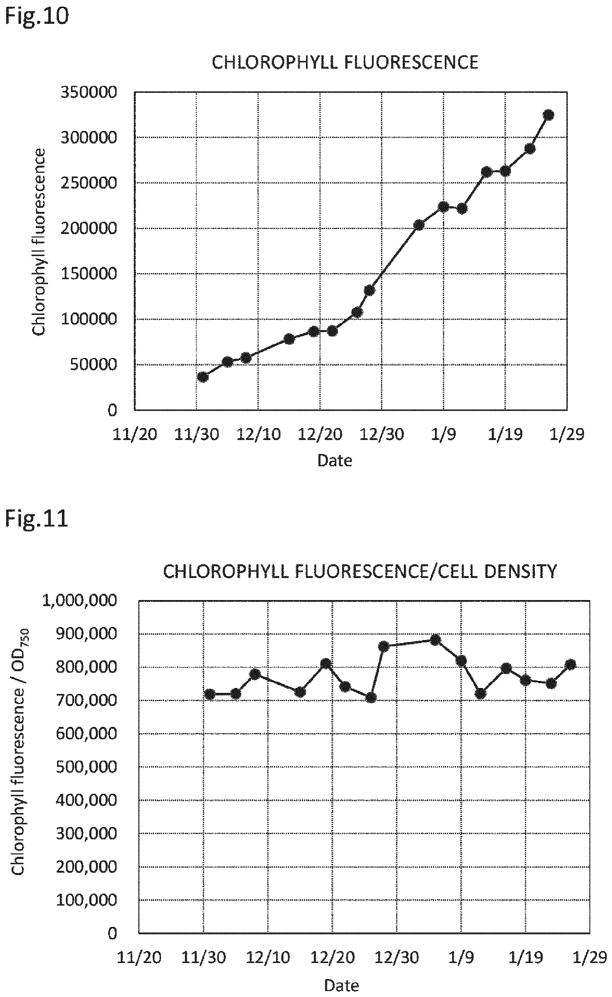

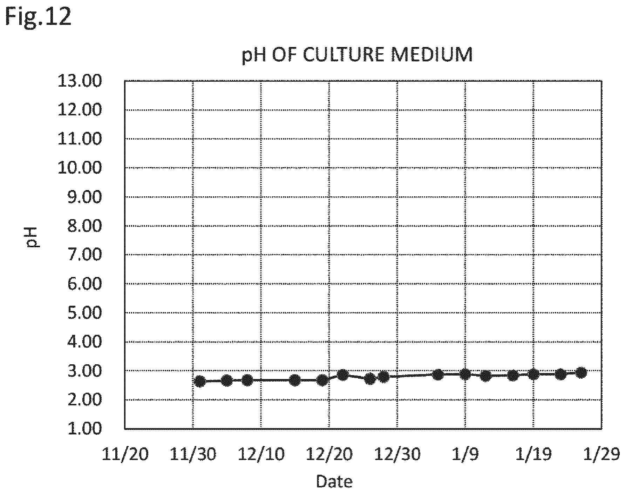

Fig. 9 is a graph showing the results of measuring a cell density in Experiment 4. Fig. 10 is a graph showing the results of measuring chlorophyll fluorescence in Experiment 4. Fig. 11 is a graph showing the results of chlorophyll fluorescence/cell density in Experiment 4. Fig. 12 is a graph showing the results of measuring the pH of culture medium in Experiment 4. Figs. 9 to 12 show the results after pH adjustment of the culture medium by addition of dilute sulfuric acid. As shown in Figs. 9 and 10, as the culture period became longer, the chlorophyll fluorescence intensity increased and the cell density increased. Since the culture period was in winter, the temperature of the culture medium did not rise, and the growth rate of cells tended to be slightly slow. As shown in Fig. 11, the chlorophyll level divided by the cell density was kept at a substantially constant value. This indicates that the growth of other organisms is suppressed in the culture medium, and only the diatom having chlorophyll proliferates. In addition, as shown in Fig. 12, the pH of the culture medium was maintained at pH 2.5 to 3.0 over the culture period. Therefore, it has been demonstrated that the diatom in the present disclosure is obtained using a strongly acidic medium and thus useful for mass culture even in an outdoor environment in which other microorganisms, insects, and the like may be mixed.

The present invention is not limited to the above-described embodiments, and can be realized by various configurations without departing from the spirit of the present invention. For example, the technical features in the embodiments and Examples corresponding to the technical features in the respective embodiments described in the Summary of Invention section can be appropriately replaced or combined in order to solve part or all of the above-described problems or achieve part or all of the above-described effects. Besides, when the technical features are not herein described as essential, they can be deleted if appropriate.

## Claims

1. A method for producing a fucoxanthin-containing composition, the method comprising
a step of culturing a diatom in a medium at pH 1.0 or more and pH 4.0 or less, wherein
the diatom has 18S rRNA composed of the following (a) or (b):
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.

2. The method for producing a fucoxanthin-containing composition according to claim 1, wherein
the medium contains silicon corresponding to sodium metasilicate nonahydrate at 100 mg/L or more and 10 g/L or less.

3. The method for producing a fucoxanthin-containing composition according to claim 1 or 2, wherein
the pH of the medium is pH 1.5 or more and pH 2.5 or less.

4. The method for producing a fucoxanthin-containing composition according to any one of claims 1 to 3, wherein
the medium contains seawater.

5. The method for producing a fucoxanthin-containing composition according to any one of claims 1 to 4, wherein
a sodium ion concentration of the medium is 0.1 M or more and 0.6 M or less.

6. The method for producing a fucoxanthin-containing composition according to any one of claims 1 to 5, wherein
the medium is a liquid medium, and
the method further comprises, after the culturing step,
a sedimentation step of settling the diatom by allowing the liquid medium to stand; and
a step of removing the liquid medium after the sedimentation step.

7. A fucoxanthin-containing composition comprising a diatom and fucoxanthin, wherein
the diatom has 18S rRNA composed of the following (a) or (b) and is viable at pH 1.0 or more and pH 4.0 or less:
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.

8. The fucoxanthin-containing composition according to claim 7, further comprising silicon.

9. The fucoxanthin-containing composition according to claim 7 or 8, wherein
a ratio of content of the fucoxanthin to content of the diatom is 0.1 mass% or more and 5 mass% or less in terms of dry mass.

10. The fucoxanthin-containing composition according to claim 7 or 8, wherein
a ratio of content of the fucoxanthin to content of the diatom is 50 mass% or more and 99.9 mass% or less in terms of dry mass.

11. The fucoxanthin-containing composition according to any one of claims 7 to 10, wherein
the diatom is viable at pH 1.5 or more and pH 2.5 or less.

12. A food comprising the fucoxanthin-containing composition according to any one of claims 7 to 11.

13. A cosmetic comprising the fucoxanthin-containing composition according to any one of claims 7 to 11.

14. A diatom comprising 18S rRNA composed of the following (a) or (b) and being viable at pH 1.0 or more and pH 4.0 or less:
(a) a polynucleotide consisting of a nucleotide sequence represented by SEQ ID NO: 1; or
(b) a polynucleotide consisting of a nucleotide sequence having 85% or more identity to the nucleotide sequence represented by SEQ ID NO: 1.
